# EUROPEAN PATENT APPLICATION

(11) **EP 2 277 519 A2**
(43) Date of publication of application: **26.01.2011**
(21) Application number: 10010670.7
(22) Date of filing: 25.08.2000
(51) Int. Cl.: A61K 31/41, A61K 31/4178, A61K 31/4184, A61K 31/519, A61P 9/04, A61P 9/10, A61P 3/10

(54) **Use of Angiotensin II type 1 receptor antagonists for the prevention of stroke, diabetes and/or congestive heart failure**

(30) Priority: 27.08.1999 SE 9903028
(62) Divisional of application: 04006330.7
(71) Applicant: Sanofi-Aventis Deutschland GmbH, 65929 Frankfurt am Main (DE)
(72) Inventor: Schoelkens, Bernward, Prof., Dr., 65926 Frankfurt am Main (DE); Bender, Norbert, Dr., 67098 Bad Dürckheim (DE); Rangoonwala, Badrudin, Dr., 65926 Frankfurt am Main (DE); Dagenais, Gilles, St. Nicolas Quebec, J7A 3P8 (CA); Gerstein, Hertzel, Hamilton Ontario L8S 4A8 (CA); Ljunggren, Anders, 43163 Mölndal (SE); Yusuf, Salim, Carlise Ontario L0R 1H2 (CA)

(57) **Abstract**

The present invention relates to use of an inhibitor of the renin-angiotensin system (RAS) or a pharmaceutically acceptable derivative thereof, particularly ramipril or ramiprilat, in the manufacture of a medicament for the prevention of stroke, diabetes and /or congestive heart failure (CHF). The present invention further relates to a method of prevention and/or treatment of stroke, diabetes and/or CHF, comprising administering a therapeutically effective amount of an inhibitor of the RAS or a pharmaceutically acceptable derivative thereof, particularly ramipril or ramiprilat, to a patient in need of such prevention and/or treatment.

## Description

### FIELD OF INVENTION

The present invention relates to use of an inhibitor of the renin-angiotensin system (RAS) or a pharmaceutically acceptable derivative thereof in the manufacture of a medicament for the prevention of stroke, diabetes and /or congestive heart failure (CHF). The present invention further relates to a method of prevention and/or treatment of stroke, diabetes and/or CHF, comprising administering a therapeutically effective amount of an inhibitor of the RAS or a pharmaceutically acceptable derivative thereof to a patient in need of such prevention and/or treatment.

### BACKGROUND OF THE INVENTION

Compounds that interfere with the RAS are well known in the art and are used to treat cardiovascular diseases, particularly arterial hypertension and heart failure. Principally, the RAS can be interferred with by inhibition of the enzymes synthesizing angiotensins or by blocking the corresponding receptors at the effector sites. Available today are inhibitors of the angiotensin converting enzyme (ACE) and angiotensin II type 1 receptor (AT II) antagonists.

ACE inhibitors are compounds which inhibit the conversion of angiotensin I into the active angiotensin II as well as the breakdown of the active vasodilator bradykinin. Both of these mechanisms lead to vasodilation. Such compounds have been described in, for example, EP 158927, EP 317878, US 4,743,450, and US 4,857,520.

Ramipril (disclosed in EP-A-079022) is a long-acting ACE inhibitor. Its active metabolite is the free diacid ramiprilat, which is obtained in vivo upon administration of ramipril. In hypertensive patients administration of ramipril is known to cause a reduction in peripheral arterial resistance and thus a reduction of the blood pressure without a compensatory rise in heart rate. It is currently being used in the treatment of hypertension and CHF. Furthermore, ramipril has been shown to reduce mortality in patients with clinical signs of congestive heart failure after surviving an acute myocardial infarction. Ramipril has been suggested to have an added advantage over many other ACE inhibitors due to its pronounced inhibition of ACE in tissues resulting in organ protective effects in e.g. the heart, kidney, and blood vessels.

Compounds that interfere with the RAS including ACE inhibitors and AT II antagonists are currently used in the treatment of various cardiovascular disorders, especially in patients exhibiting a high blood pressure. Use of said compounds in prevention of cardiovascular disorders is much less common and the use of said compounds in the prevention of stroke, diabetes and /or CHF is hitherto unknown.

### SUMMARY OF THE INVENTION

The present invention relates to use of an inhibitor of the RAS or a pharmaceutically acceptable derivative thereof in the manufacture of a medicament for the prevention of stroke, especially in patients exhibiting normal or low blood pressure.

The present invention further relates to use of an inhibitor of the RAS or a pharmaceutically acceptable derivative thereof in the manufacture of a medicament for the prevention of diabetes.

The present invention also relates to use of an inhibitor of the RAS or a pharmaceutically acceptable derivative thereof in the manufacture of a medicament for the prevention of development of CHF in patients with no preexisting CHF, i.e. no signs or symptoms of CHF.

Another aspect of the invention is a method of prevention of stroke, diabetes and/or CHF, comprising administering a therapeutically effective amount of an inhibitor of the RAS or a pharmaceutically acceptable derivative thereof, to a patient in need of such prevention.

Yet another aspect of the invention is a pharmaceutical formulation for use in the prevention of stroke, diabetes and/or CHF, comprising a therapeutically effective amount of an inhibitor of the RAS or a pharmaceutically acceptable derivative thereof.

A further aspect of the invention is the use of an inhibitor of the RAS or a pharmaceutically acceptable derivative thereof, in the prevention of stroke, diabetes and/or CHF, by administering the inhibitor of the RAS or a pharmaceutically acceptable derivative thereof, to a patient in need of such prevention.

### DETAILED DESCRIPTION OF THE INVENTION

It has surprisingly been found that cardiovascular and metabolic disorders such as stroke, diabetes and CHF can be prevented by use of an inhibitor of RAS, particularly an ACE inhibitor that interferes with the synthesis of angiotensin II. The present invention is especially surprising in that especially patients with an essentially maintained heart function and/or exhibiting a normal or low blood pressure benefit markedly from the preventive action of the inhibitors of RAS. The invention describes a new method to prevent disorders such as stroke, diabetes and/or CHF by administration of an inhibitor of the RAS.

Patients exhibiting a normal or low blood pressure are known as normotensive patients. Examples of guidelines defining blood pressure values for different patient groups including different ages, include guidelines issued by the WHO and JNC (USA). In the present invention, a suitable definition of a normal or low blood pressure can be found in JNC VI, which is hereby incorporated by reference.

In the present invention, "stroke" includes both fatal and non-fatal.

In the present invention, "diabetes" include both type I diabetes, also known as insulin-dependent, diabetes mellitus (IDMM), and type II diabetes, also known as non-insulin-dependent diabetes mellitus (NIDDM).

In the present invention, "inhibitor of the renin-angiotensin system (RAS) or a pharmaceutically acceptable derivative thereof" includes any compound which in itself or upon administration blocks the negative effects of angiotensin II on the vasculature either by reducing the synthesis of angiotensin II or blocking its effect at the receptor.

In the present invention, "angiotensin converting enzyme (ACE) inhibitor or a pharmaceutically acceptable derivative thereof" includes any compound which in itself or upon administration interferes with the synthesis of angiotensin II.

When the inhibitor of the RAS used in the present invention have several asymetric carbon atoms, they can consequently exist in several stereochemical forms. The present invention includes the mixture of isomers as well as the individual stereoisomers. The present invention further includes geometrical isomers, rotational isomers, enantiomers, racemates and diastereomers.

Where applicable, the inhibitors of RAS may be used in neutral form, e.g. as a carboxylic acid, or in the form of a salt, preferably a pharmaceutically acceptable salt such as the sodium, potassium, ammonium, calcium or magnesium salt of the compound at issue. Where applicable the compounds listed above can be used in hydrolyzable ester form.

In the present invention, the inhibitors of the RAS include all prodrugs thereof, whether active or inactive *in vitro.* Thus, although such protected derivatives may not possess pharmacological activity *per se*, they may be administered e.g. parenterally or orally, and thereafter metabolized *in vivo* to form pharmacologically active inhibitors of RAS. Preferred examples are ramipril, which is metabolized into ramiprilat, and candesartan cilexetil, which is metabolized into candesartan. Inhibitors of the RAS include ACE inhibitors, AT II antagonists, also known as angiotensin receptor blockers (ARBs), renin antagonists, and vasopeptidase inhibitors (VPIs).

The phrase "vasopeptidase inhibitors" embraces so-called NEP/ACE inhibitors (also referred to as selective or dual acting neutral endopeptidase inhibitors) which possess neutral endopeptidase (NEP) inhibitory activity and angiotensin converting enzyme (ACE) inhibitory activity.

The phrase "renin antagonists" embraces renin inhibitors.

In the present invention, the RAS inhibitors may exhibit a long term duration, medium term duration or short term duration.

ACE inhibitors or pharmaceutically acceptable derivatives thereof, including active metabolites, which can be used for the prevention of stroke, diabetes and/or CHF include, but is not limited to, the following compounds:
alacepril, alatriopril, altiopril calcium, ancovenin, benazepril, benazepril hydrochloride, benazeprilat, benzoylcaptopril, captopril, captopril-cysteine, captopril-glutathione, ceranapril, ceranopril, ceronapril, cilazapril, cilazaprilat, delapril, delapril-diacid, enalapril, enalaprilat, enapril, epicaptopril, foroxymithine, fosfenopril, fosenopril, fosenopril sodium, fosinopril, fosinopril sodium, fosinoprilat, fosinoprilic acid, glycopril, hemorphin-4, idrapril, imidapril, indolapril, indolaprilat, libenzapril, lisinopril, lyciumin A, lyciumin B, mixanpril, moexipril, moexiprilat, moveltipril, muracein A, muracein B, muracein C, pentopril, perindopril, perindoprilat, pivalopril, pivopril, quinapril, quinapril hydrochloride, quinaprilat, ramipril, ramiprilat, spirapril, spirapril hydrochloride, spiraprilat, spiropril, spiropril hydrochloride, temocapril, temocapril hydrochloride, teprotide, trandolapril, trandolaprilat, utibapril, zabicipril, zabiciprilat, zofenopril and zofenoprilat.

Preferred ACE inhibitors for use in the present invention are ramipril, ramiprilat, lisinopril, enalapril and enalaprilat. More preferred ACE inhibitors for uses in the present invention are ramipril and ramiprilat. Information about ramipril and ramiprilat can be obtained e.g. from the Merck Index., 12th ed., 1996, pp. 1394-1395.

AT II antagonists or pharmaceutically acceptable derivatives thereof, including active metabolites, which can be used for the prevention of stroke, diabetes and/or CHF include, but is not limited to, those described in European Patent Applications, Publication Nos. 253310, 323841, 324377, 399731, 400974, 401030, 403158, 403159, 407102, 407342, 409332, 411507, 411766, 412594, 412848, 415886, 419048, 420237, 424317, 425211, 425921, 426021, 427463, 429257, 430300, 430709, 432737, 434038, 434249, 435827, 437103, 438869, 442473, 443568, 443983, 445811, 446062, 449699, 450566, 453210, 454511, 454831, 456442, 456442, 456510, 459136, 461039, 461040, 465323, 465368, 467207, 467715, 468372, 468470, 470543, 475206, 475898, 479479, 480204, 480659, 481448, 481614, 483683, 485929, 487252, 487745, 488532, 490587, 490820, 492105, 497121,497150,497516,498721,498722,498723,499414,499415,499416, 500297, 500409, 501269, 501892, 502314, 502575, 502725, 503162, 503785, 503838, 504888, 505098, 505111, 505893, 505954, 507594, 508393, 508445, 508723, 510812, 510813, 511767, 511791, 512675, 512676, 512870, 513533, 513979, 514192, 514193, 514197, 514198, 514216, 514217, 515265, 515357, 515535, 515546, 515548, 516392, 517357, 517812, 518033, 518931, 520423, 520723, 520724, 521768, 522038, 523141, 526001, 527534, and 528762. Other AII antagonists include those disclosed in International Patent Application, Publication Nos. WO 91/00277, WO 91/00281, WO 91/11909, WO 91/11999, WO 91/12001, WO 91/12002, WO 91/13063, 91/15209, WO 91/15479, WO 91/16313, WO 91/17148, WO 91/18888, WO 91/19697, WO 91/19715, WO 92/00067, WO 92/00068, WO 92/00977, WO 92/02510, WO 92/04335, WO 92/04343, WO 92/05161, WO 92/06081, WO 92/07834, WO 92/07852, WO 92/09278, WO 92/09600, WO 92/10189, WO 92/11255, WO 92/14714, WO 92/16523, WO 92/16552, WO 92/17469, WO 92/18092, WO 92/19211, WO 92/20651, WO 92/20660, WO 92/20687, WO 92/21666, WO 92/22533, WO 93/00341, WO 93/01177, WO 93/03018, WO 93/03033 and WO 93/03040. The contents of the aforesaid European and International Patent Applications are hereby incorporated by reference thereto.

Preferred AT II antagonists or pharmaceutically acceptable derivatives thereof for use in the present invention include, but is not limited to, compounds with the following generic names: candesartan, candesartan cilexetil, losartan, valsartan, irbesartan, tasosartan, telmisartan and eprosartan.

Particularly preferred AT II antagonists or pharmaceutically acceptable derivatives thereof for use in the present invention are candesartan and candesartan cilexetil. Candesartan and candesartan cilexetil are known from European Patent No. 459 136 B1, US 5,196,444 and US 5,703,110 to Takeda Chemical Industries. Candesartan cilexetil is currently manufactured and sold world-wide by AstraZeneca and Takeda.e.g. under the trade names Atacand^{®}, Amias^{®} and Blopress^{®}.

NEP/ACE-inhibitors or pharmaceutically acceptable derivatives thereof, including active metabolites, which can be used for the prevention of stroke, diabetes and/or CHF include, but is not limited to, those compounds disclosed in U.S. Patents Nos. 5,508,272, 5,362,727, 5,366,973, 5,225,401, 4,722,810, 5,223,516, 5,552,397, 4,749,688, 5,504,080, 5,612,359, 5,525,723, 5,430,145, and 5,679,671, and European Patent Applications 0481522, 0534263, 0534396, 0534492 and 0671172.

Preferred NEP/ACE inhibitors for use in the present invention are those which are designated as preferred in the above U.S. patents and European Patent Applications and are incorporarted herein by reference. Especially preferrred is the NEP/ACE inhibitor omapatrilat (disclosed in U.S. Patent No. 5,508,272), or MDL100240 (disclosed in U.S. Patent No. 5,430,145).

Renin-inhibitors or pharmaceutically acceptable derivatives thereof, including active metabolites, which can be used for the prevention of stroke, diabetes and/or CHF include, but is not limited to, the following compounds: enalkrein; RO 42-5892; A 65317; CP 80794; ES 1005; ES 8891; SQ 34017; CGP 29287; CGP 38560; SR 43845; U-71038; A 62198; and A 64662.

### Pharmaceutical formulations

In one aspect, the present invention relates to pharmaceutical formulations comprising as active ingredient an RAS inhibitor or a pharmaceutically acceptable derivative or prodrug thereof, including metabolites, for use in the prevention of stroke, diabetes and/or conge-stive heart failure (CHF).

For clinical use, the RAS inhibitor is formulated into a pharmaceutical formulation for oral, intravenous, subcutaneous, tracheal, bronchial, intranasal, pulmonary, transdermal, buccal, rectal, parenteral or some other mode of administration. The pharmaceutical formulation may contain the inhibitor in admixture with a pharmaceutically acceptable adjuvant, diluent and/or carrier.

In the preparation of the pharmaceutical formulations of the present invention the active ingredient may be mixed with solid, powdered ingredients, such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or pressed into tablets.

The active ingredient may be separately premixed with the other, non-active ingredients, before being mixed to form a formulation.

Soft gelatine capsules may be prepared with capsules containing a mixture of the active ingredient of the invention, vegetable oil, fat, or other suitable vehicle for soft gelatine capsules. Hard gelatine capsules may contain granules of the active ingredients. Hard gelatine capsules may also contain the active ingredients in combination with solid powdered ingredients such as lactose, saccharose, sorbitol, mannitol, potato starch, corn starch, amylopectin, cellulose derivatives or gelatine.

Dosage units for rectal administration may be prepared (i) in the form of suppositories which contain the active substance mixed with a neutral fat base; (ii) in the form of a gelatine rectal capsule which contains the active substance in a mixture with a vegetable oil, paraffin oil or other suitable vehicle for gelatine rectal capsules; (iii) in the form of a ready-made micro enema; or (iv) in the form of a dry micro enema formulation to be reconstituted in a suitable solvent just prior to administration.

Liquid preparations may be prepared in the form of syrups or suspensions, e.g. solutions or suspensions containing the active ingredients and the remainder consisting, for example, of sugar or sugar alcohols and a mixture of ethanol, water, glycerol, propylene glycol and polyethylene glycol. If desired, such liquid preparations may contain coloring agents, flavoring agents, preservatives, saccharine and carboxymethyl cellulose or other thickening agents. Liquid preparations may also be prepared in the form of a dry powder to be recon-stituted with a suitable solvent prior to use.

Solutions for parenteral administration may be prepared as a solution of a formulation of the invention in a pharmaceutically acceptable solvent. These solutions may also contain stabilizing ingredients, preservatives and/or buffering ingredients. Solutions for parenteral administration may also be prepared as a dry preparation to by reconstituted with a suitable solvent before use.

The total amount of active ingredient suitably lies in the range of from about 0.1 % (w/w) to about 95 % (w/w) of the formulation, suitably from 0.5 % to 50 % (w/w) and preferably from 1 % to 25 % (w/w).

The pharmaceutical formulations may contain between about 0.1 mg and about 1000 mg of active ingredient, preferably between 1 mg and 100 mg of active ingredient. The dose of the active ingredient to be administered will depend on the relevant indication, the age, weight and sex of the patient and may be determined by a physician. The dosage will suitably be in the range of from about 0.01 mg/kg to about 20 mg/kg, preferably between 0.1 mg/kg and 10 mg/kg.

The typical daily dose of the active ingredients varies within a wide range and will depend on various factors such as the relevant indication, the route of administration, the age, weight and sex of the patient and may be determined by a physician. In general, dosages, and especially oral and parenteral dosages, will be in the range of from about 0.1 to about 100 mg per day of active ingredient, preferably between 1 and 50 mg per day of active ingredient.

The following Example is intended to illustrate, but in no way limit the scope of the invention.

### EXAMPLE

A large-scale clinical trial was designed to examine the effect of the ACE inhibitor ramipril versus placebo in reducing cardiovascular events.

The study was conducted in 267 centres in 19 countries over a six year period and included 9,541 participants who are at high risk for cardiovascular events due to a history of previous ischaemic heart disease, stroke, peripheral arterial disease or individuals with diabetes.

The systolic blood pressure at inclusion of the patients was on average 138 mm Hg and thus the patients were normotensive at study start. After one month of therapy with either ramipril or placebo, the systolic blood pressure had decreased by 5.48 mm Hg and 1.59 mm Hg, respectively.

The primary endpoint of the study was myocardial infarction (MI), stroke and cardiovascular (CV) death (mortality).

The study was stopped early because of a very clear reduction in the combined endpoint of cardiovascular deaths, heart attacks and strokes in patients taking ramipril. In addition to the above benefits, there was also a reduction of between a fourth and a fifth in the need for revascularisation procedures (such as coronary artery bypass graft surgery, balloon angioplasty, etc.) and diabetic complications.

There was a clear 32% reduction in the ramipril group in the number of patients who developed a stroke, and this is surprising since patients were normotensive when recruited to the study.

The number of patients who developed CHF was significantly reduced by 21% in the ramipril group, which is unexpected since patients had no signs or symptoms of CHF at study start.

Equally surprising is the marked 36% reduction in the number of patients who developed diabetes in the ramipril group.

### Abbrevations

ACE = angiotensin converting enzyme
AT II = angiotensin II type 1 receptor
CHF = congestive heart failure
IDMM = insulin-dependent, diabetes mellitus
JNC = Joint National Committee
MI = myocardial infarction
NIDDM = non-insulin-dependent diabetes mellitus
WHO = World Health Organization

## Claims

1. The use of an angiotensin converting enzyme inhibitor or a pharmaceutically acceptable salt thereof in the manufacture of a medicament for the prevention of stroke.

2. The use of an angiotensin converting enzyme inhibitor or a pharmaceutically acceptable salt thereof in the manufacture of a medicament for the prevention of stroke in patients exhibiting normal or low blood pressure.

3. The use of an inhibitor an angiotensin converting enzyme inhibitor or a pharmaceutically acceptable salt thereof in the manufacture of a medicament for the prevention of diabetes.

4. The use of an inhibitor of an angiotensin converting enzyme inhibitor or a pharmaceutically acceptable salt thereof in the manufacture of a medicament for the prevention of development of congestive heart failure (CHF) in patients with no preexisting CHF.

5. The use according to any one of claims 1-4, wherein the ACE inhibitor or a pharmaceutically acceptable salt thereof is selected from the group consisting of alacepril, alatriopril, altiopril calcium, ancovenin, benazepril, benazepril hydrochloride, benazeprilat, benzoylcaptopril, captopril, captopril-cysteine, captopril-glutathione, ceranapril, ceranopril, ceronapril, cilazapril, cilazaprilat, delapril, delapril-diacid, enalapril, enalaprilat, enapril, epicaptopril, foroxymithine, fosfenopril, fosenopril, fosenopril sodium, fosinopril, fosinopril sodium, fosinoprilat, fosinoprilic acid, glycopril, hemorphin-4, idrapril, imidapril, indolapril, indolaprilat, libenzapril, lisinopril, lyciumin A, lyciumin B, mixanpril, moexipril, moexiprilat, moveltipril, muracein A, muracein B, muracein C, pentopril, perindopril, perindoprilat, pivalopril, pivopril, quinapril, quinapril hydrochloride, quinaprilat, ramipril, ramiprilat, spirapril, spirapril hydrochloride, spiraprilat, spiropril, spiropril hydrochloride, temocapril, temocapril hydrochloride, teprotide, trandolapril, trandolaprilat, utibapril, zabicipril, zabiciprilat, zofenopril and zofenoprilat.

6. The use according to claim 5, wherein the ACE inhibitor is selected from the group consisting of ramipril, ramiprilat, lisinopril, enalapril and enalaprilat.

7. The use according to claim 6, wherein the ACE inhibitor is selected from the group consisting of ramipril or ramiprilat.

8. A method of prevention of stroke, comprising administering a therapeutically effective amount of an angiotensin converting enzyme inhibitor or a pharmaceutically acceptable salt thereof to a patient in need of such prevention.

9. The method of prevention of stroke in patients exhibiting normal or low blood pressure, comprising administering a therapeutically effective amount of an angiotensin converting enzyme inhibitor or a pharmaceutically acceptable salt thereof, to a patient in need of such prevention.

10. The method of prevention of diabetes, comprising administering a therapeutically effective amount of an angiotensin converting enzyme inhibitor or a pharmaceutically acceptable salt thereof, to a patient in need of such prevention.

11. The method of prevention of development of CHF in patients with no preexisting CHF, comprising administering a therapeutically effective amount of an angiotensin converting enzyme inhibitor or a pharmaceutically acceptable salt thereof, to a patient in need of such prevention.

12. The method of prevention according to any one of claims 8 to 11, wherein the ACE inhibitor or a pharmaceutically salt derivative thereof is selected from the group consisting of alacepril, alatriopril, altiopril calcium, ancovenin, benazepril, benazepril hydrochloride, benazeprilat, benzoylcaptopril, captopril, captopril-cysteine, captopril-glutathione, ceranapril, ceranopril, ceronapril, cilazapril, cilazaprilat, delapril, delapril-diacid, enalapril, enalaprilat, enapril, epicaptopril, foroxymithine, fosfenopril, fosenopril, fosenopril sodium, fosinopril, fosinopril sodium, fosinoprilat, fosinoprilic acid, glycopril, hemorphin-4, idrapril, imidapril, indolapril, indolaprilat, libenzapril, lisinopril, lyciumin A, lyciumin B, mixanpril, moexipril, moexiprilat, moveltipril, muracein A, muracein B, muracein C, pentopril, perindopril, perindoprilat, pivalopril, pivopril, quinapril, quinapril hydrochloride, quinaprilat, ramipril, ramiprilat, spirapril, spirapril hydrochloride, spiraprilat, spiropril, spiropril hydrochloride, temocapril, temocapril hydrochloride, teprotide, trandolapril, trandolaprilat, utibapril, zabicipril, zabiciprilat, zofenopril and zofenoprilat.

13. The method of prevention according to claim 12, wherein the ACE inhibitor is selected from the group consisting of ramipril, ramiprilat, lisinopril, enalapril and enalaprilat.

14. The method according to claim 13, wherein the ACE inhibitor is selected from the group consisting of ramipril, or ramiprilat.

15. The use of an angiotensin converting enzyme inhibitor or a pharmaceutically acceptable salt thereof in the the prevention of stroke, diabetes and/or CHF, by administering the angiotensin converting enzyme inhibitor or a pharmaceutically acceptable salt thereof to a patient in need of such prevention.

16. The use according to claim 15, wherein the ACE inhibitor is selected from the group consisting of ramipril, ramiprilat, lisinopril, enalapril and enalaprilat.

17. The use according to claim 16, wherein the ACE inhibitor is selected from the group consisting of ramipril, or ramiprilat.
